# EUROPEAN PATENT SPECIFICATION

(11) **EP 0 822 195 B1**
(45) Date of publication and mention of the grant of the patent: **13.10.1999**
(21) Application number: 97112215.5
(22) Date of filing: 17.07.1997
(51) Int. Cl.: C07D 501/06, C07D 501/36

(54) **A process for the preparation of cephalosporins**
Verfahren zur Herstellung von Cephalosporinen
Procédé pour la préparation de céphalosporines

(30) Priority: 30.07.1996 IT MI961628
(43) Date of publication of application: 04.02.1998
(73) Proprietor: Ribbon S.r.L., 20145 Milano (IT)
(72) Inventor: Fortunato, Guiseppe, 20145 Milano (IT); Maruzzelli, Gennaro, 20145 Milano (IT); Boni, Riccardo, 20145 Milano (IT); Verzini, Massimo, 20145 Milano (IT)
(74) Representative: Minoja, Fabrizio, Dr.

(56) References cited:
- EP-A- 0 156 771
- US-A- 4 159 373
- US-A- 5 625 058

## Description

### Field of the invention

The present invention relates to a process for the preparation of cephalosporins, particularly for the preparation of 7-(α-hydroxyacylamino)-3-(1-sulfoalkyltetrazol-5-yl-thiomethyl)-3-cephem-4-carboxylic acids and the salts thereof. The method provides the direct recovery, by freeze-drying, of the active ingredient in a pure form ready for the pharmaceutical use.

### Prior art

7-(α-Hydroxyacylamino)-3-(1-sulfoalkyl-tetrazol-5-yl-thiomethyl)-3-cephem-4-carboxylic acids are cephalosporanic antibiotics having a marked antibacterial activity.

These cephalosporins are the object of a number of patents, such as US 4,159,373, US 4,048,311 and US 4,093,723.

Among all the derivatives object of these patents, Cefonicid, which is commercially available in the form of the freeze-dried disodium salt, is particularly important due to its pharmaceutical activity.

A first known method for the preparation of Cefonicid involves the acylation of 7ACA (7-aminocephalosporanic acid) with a protected acylating agent, the subsequent condensation with the suitable tetrazolethiol and the final deprotection.

US Patent 4,159,373 discloses a series of processes in which 7-(α-hydroxyacylamino)-3-(1-sulfoalkyltetrazol-5-yl-thiomethyl)-3-cephem-4-carboxylic acids are prepared reacting 7-a-hydroxyacylamino-cephalosporanic acids with a tetrazolethiol in water in the presence of sodium bicarbonate. The reaction products are recovered passing the aqueous solutions, after acidification, through ion exchange resins and precipitating the sodium salts with sodium methoxide in methanol, or by means of chromatography through ion exchange resins, purification by precipitation in alcohol solvents and freeze-drying.

Another known method involves the reaction of the desired tetrazolethiol with the 7ACA 7-formamide derivative, the subsequent deprotection and the reaction with the suitably protected acylating agent.

In addition, US patents 4,159,373 and 4,576,937 already mentioned disclose the reaction of 7-amino-3-(1-sulfoalkyl-tetrazol-5-yl-thiomethyl)-3-cephem-4-carboxylic acids with D-mandelic acid chloride formyl ester in dry dimethylformamide in the presence of triethylamine and the subsequent deformylation in aqueous medium both base- and acid- catalysed.

WO 92/17600 discloses the reaction of the suitable cephalosporanic ring with O-formylmandeloyl chloride in water in the presence of sodium bicarbonate and the subsequent enzymatic deformylation.

EP-A-0156771 describes the preparation of 7-(α-hydroxyacylamino)-3-(1-sulfoalkyl-tetrazol-5-yl-thiomethyl)-3-cephem-4-carboxylic acids by reaction of the suitable cephalosporanic moiety with D-mandelic acid thioesters and the subsequent recovery of the formylated disodium salts, which are then transformed into the corresponding acid by a step through acid ion exchange resins, retransformed into the monosodium salts with sodium 2-ethylhexanoate and deformylated with concentrated hydrochloric acid in methanol.

Finally the end product is precipitated from the reaction mixture as the disodium salt upon addition of sodium hydroxide and isopropanol.

A more recent Italian patent application, n. MI93A001580, analogously to what already described in US Patent 4,159,373, reports the preparation of Cefonicid starting from 7-amino-3-(1-sulfoalkyl-tetrazol-5-yl-thiomethyl)-3-cephem-4-carboxylic acid by reaction with D-mandeloylformyl chloride and subsequent deprotection in hydrochloric acid.

The recovery of the product is effected extracting the acid in an organic solvent, concentrating the organic phase and precipitating disodium Cefonicid with sodium ethylhexanoate in an acetone-ethanol mixture.

The methods described in US 4,048,311, US 4,093,723 and 4,159,373 involve processes for the separation of the final product which are complex and difficult to practice industrially.

The more recent WO 92/17600 and EP-A-0 156 771 disclose preparation methods easier than those of the prior patents, but also in this case the recovery of the final product is still complex, yielding coloured mixtures and difficult to carry out industrially.

Italian patent application n. MI93A001580 discloses the preparation of Cefonicid following what already described in the cited patents, but partly simplifying the recovery of the final product.

The recovery of Cefonicid is effected by extraction of the acid with an organic solvent, subsequent concentration of the organic phase and precipitation of disodium Cefonicid with sodium ethylhexanoate in an acetone-ethanol mixture. This method solves only partially the problems due to the formation of coloured mixtures, in that the concentration of the final organic solution containing the product is time-consuming and complex. It should also be taken into account that the solvent used to extract Cefonicid and subsequently concentrated under vacuum is tetrahydrofuran, which is known to be dangerous due to the possible formation of peroxides.

Moreover disodium Cefonicid, which is obtained by precipitation from the acetone-ethanol mixture, has a high content in residual solvents which remain in significant amounts in the final product even after the subsequent freeze-drying step.

### Summary

The Applicant has found a process which is easy to carry out on industrial scale and overcomes the above mentioned drawbacks for the preparation of 7-α-hydroxyacylamino cephalosporins of formula (I) 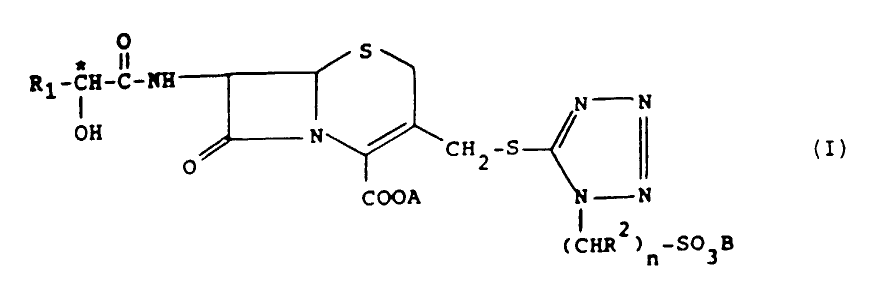 wherein
- R₁ is a phenyl group, optionally substituted with 1 to 3 substituents selected from halogens and hydroxyl groups, a heterocyclic ring of 4 to 7 atoms, both aromatic and non aromatic or a group selected from dihydrophenyl, cyclohexyl, hydroxymethyl, formamido, amido and carboxymethylamino;
- R₂ is hydrogen or a straight or branched alkyl group having 1 to 12 carbon atoms;
- n is an integer of 1 to 10;
- A and B, which are the same or different, are pharmaceutically acceptable cations, for example cations of alkali or alkaline-earth metals such as sodium, potassium and calcium or cations such as ammonium or the cations of organic amines.

The compounds of formula (I) have an asymmetric carbon atom in the side chain at the 7- position of the "cephem" ring and therefore can exist as the D- or L-forms or as the mixtures thereof.

The process of the invention comprises the following steps:
a) condensation of 7-amino-3-cephem-4-carboxylic acid of formula (II) 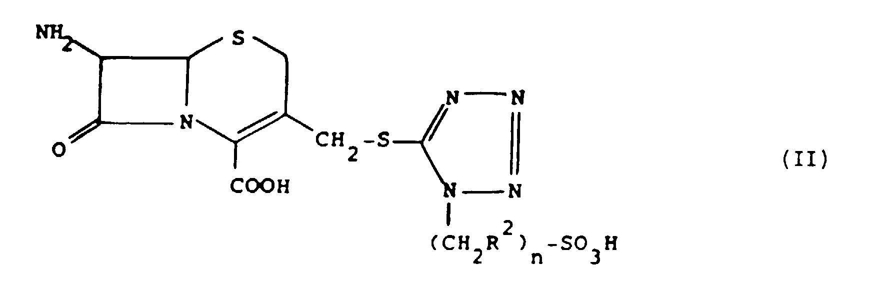 in which R₂ and n have the meaning defined above, with the formyl derivative of formula (III) 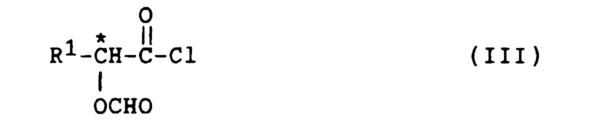 wherein R₁ is as defined above;
   the reaction being carried out in an organic or aqueous solvent or in mixtures thereof at pH ranging from 6 to 7.0 by addition of proton acceptors;
b) deformylation of the reaction product in aqueous phase by treatment with an acid at pH 0.5-1.0 to give a product of formula (I) 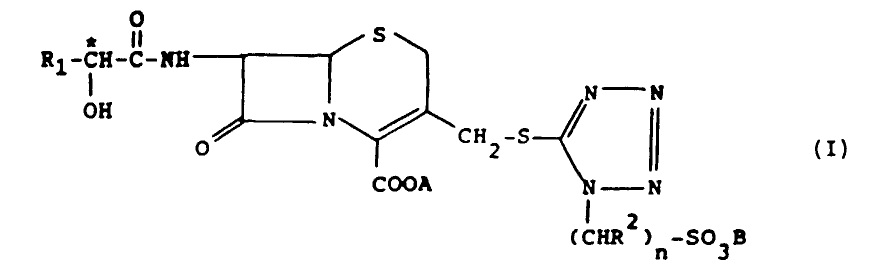
c) adjustment of the aqueous solution to pH ranging from 1.5 to 6.0 with bases containing sodium and osmotic filtration of the solution to remove the excess sodium chloride;
d) optional adjustment to pH 5.2 with bases containing sodium and freeze-drying of the solution. According to a preferred aspect of the invention, the product obtained from step b) is recovered with a process which comprises:
e) extraction of the acid product in an organic solvent by salting of the aqueous phase;
f) adjustment of the organic phase to pH ranging from 1.5 and 2.5 with bases containing sodium, filtration of the solution and re-extraction of the product in the aqueous phase;
g) adjustment of the aqueous solution to pH ranging from 1.5 to 6.0 with bases containing sodium and osmotic filtration of the solution to remove the excess sodium chloride;
h) optional adjustment to pH 5.2 with bases containing sodium and freeze-drying of the solution.

### Detailed disclosure

The method of the invention will now be described in more detail specifically referring to Cefonicid.

The intermediates used in the preparation of the compounds of formulae (II) and (III) are known, commercially available or anyhow they can easily be prepared with processes described in literature.

The first step comprises the acylation of 7-amino-3-(1-sulfoalkyl-tetrazol-5-yl-thiomethyl)-3-cephem-4-carboxylic acid with D-mandeloylformyl chloride.

The reaction can be performed in water, in a solvent or in a water-solvent mixture. The solvents used are selected from acetone, methyl acetate, tetrahydrofuran, acetonitrile, dimethylacetamide.

The proton acceptor is an inorganic or organic base or a mixture thereof, preferably NaHCO₃, Na₂CO₃, NH₃, NaOH, triethylamine, tetramethylguanidine or diazobicycloundecene.

The acylation reaction is performed in a range of temperatures from -5°C to +40°C, preferably from 0° to +10°C.

The reaction is carried out preferably in a water/acetone mixture in 70/30 to 30/70 ratio, using sodium bicarbonate as proton acceptor.

The acylation conditions are very mild and perfectly reproducible and avoid the formation of any side-products.

This way, clear solutions can be obtained from which a high quality product can be extracted.

In the second step acetone is removed from the reaction medium and the deformylation is performed in an acid aqueous medium. Acetone can be removed either by concentration under vacuum at temperatures from 20° to 40°C or by extraction from the aqueous phase with solvents such as toluene, ethyl acetate, methyl isobutyl ketone, methylene chloride or chloroform.

In the reaction conditions, extraction with toluene can be carried out.

The acetone residual content in the aqueous phase after extraction usually ranges from 1 to 5%.

The aqueous solution containing the formylated salified product is added with an acid to adjust pH to a value ranging from 0.5 to 1.0. The deformylation is performed at a temperature from +15°C to +30°C for a time ranging from 20 to 48 hours.

The acid used for the deformylation can be inorganic, such as hydrochloric acid, sulfuric acid or phosphoric acid or organic, such as trifluoromethanesulfonic or methanesulfonic acids.

Preferably, hydrochloric acid at pH = 0.5 is used at 25°C for a time of 20 hours.

The deformylation conditions too are particularly mild and highly selective, thus allowing to significantly decrease the formation of undesired by-products.

The final deformylation mixture contains almost only Cefonicid and salts in aqueous solution.

In the subsequent step, pH of the aqueous solution is adjusted to values from 1.5 to 6.0 with bases containing sodium such as NaHCO₃, Na₂CO₃, NaOH and the solution is subjected to reverse osmosis by filtration through a nanofiltration membrane. The filtration is performed at a temperature from +5°C to +30°C at 10-40 bars. During filtration, distilled water is added to the solution containing Cefonicid to keep the starting volume constant.

At the end of the osmotic filtration, two solutions are obtained, one rich in Cefonicid and the other in salts.

The solution containing Cefonicid at a concentration varying from 15% to 50% is adjusted to pH from 4.5 to 6.0 with an inorganic base selected from NaHCO₃, Na₂CO₃ and NaOH, filtered over charcoal, through a sterilizing 0.22 µm membrane and finally frozen at a temperature from -30°C to -60°C.

The frozen solution is freeze-dried under a vacuum ranging from 10 to 400 microns, with a heating temperature from +10°C to +70°C.

The freeze-drying process yields highly pure disodium Cefonicid, ready for the pharmaceutical use.

The used acylation and deformylation conditions are rather mild and give a solution containing almost only Cefonicid and salts. The subsequent conditions of osmotic filtration are also mild and allow to separate the pure Cefonicid in aqueous solution from the salts, thereby obtaining, by means of freeze-drying, a highly pure product ready for the pharmaceutical use and free from residual solvents such as acetone and alcohols.

The solvents used in some prior art processes to recover Cefonicid disodium salt are difficult to remove either drying the product under vacuum or freeze-drying it in aqueous solution.

The procedure used in this invention avoids the recovery of the oral product by precipitation and allows to recover the product in pure form from the aqueous solution thanks to the osmotic filtration technique.

The final freeze-drying of the solution yields Cefonicid disodium salt ready for the pharmaceutical use.

In addition to the above mentioned advantages, this process is simple and particularly convenient from the industrial stand-point in that it avoids the precipitation and the recovery of the product which is highly hygroscopic and difficult to dry.

The invention also relates to the recovery of Cefonicid after the deformylation by extraction in organic solvents such as acetone, methyl acetate and acetonitrile by salting the aqueous solution.

After the deformylation, pH of the aqueous solution is adjusted to 1.5 - 6.0 with inorganic bases, an organic solvent, preferably acetone, is added and the aqueous solution is saturated with sodium chloride.

Cefonicid from the aqueous phase passes into the organic solvent, decolourizing charcoal is added to the mixture which is stirred and filtered.

In the subsequent step, the product is transferred to the aqueous solution. The organic phase containing Cefonicid is added with distilled water and the organic solvent is removed from the solution either by concentration under vacuum at temperatures from +20°C to +40°C or by extraction of the aqueous phase with water-immiscible solvents such as toluene, ethyl acetate, methyl isobutyl ketone, methylene chloride or chloroform.

In the reaction conditions, the extraction is preferably performed with toluene. This way an aqueous solution containing Cefonicid and inorganic salts is obtained.

In the subsequent step, pH of the aqueous solution is adjusted to values ranging from 1.5 to 6.0 and the solution is filtered by reverse osmosis through a nanofiltration membrane.

The filtration is performed at a temperature from +5 to +30°C and under a pressure from 10 and 40 bars.

During the filtration, the solution containing Cefonicid is added with distilled water to keep the starting volume constant.

At the end of the osmotic filtration two solutions are obtained, one rich in Cefonicid and the other in salts. The solution containing Cefonicid, at a concentration varying from 15% to 50%, is adjusted to a pH ranging from 4.5 to 6.0 with an inorganic base selected from NaHCO₃, Na₂CO₃ and NaOH, filtered through charcoal, then through a 0.22 µm sterilizing membrane and finally frozen at a temperature from -30°C to -60°C.

The frozen solution is freeze-dried under a vacuum below 400 microns, preferably from 10 to 300 microns, with a heating temperature from +10°C to +70°C. The freeze-drying process gives disodium Cefonicid with a high purity grade, ready for the pharmaceutical use.

This process also avoids the recovery of the oral product by precipitation, with the already mentioned remarkable advantages and allowing to recover highly pure disodium Cefonicid by freeze-drying.

The following examples further illustrate the invention.

### Example 1

50 g of 7-amino-3-[sulfomethyl-1-H-tetrazol-5-il)thiomethyl]-3-cephem-4-carboxylic acid are suspended in 125 ml of water and 350 ml of acetone. The mixture is cooled at +5°C and solubilized with 20 g of NaHCO₃ dissolved in 250 ml of water. The completely clear solution is added with 22 ml of D-(-)-mandeloylformyl chloride dissolved in 50 ml of acetone at +5°C ± 1°C, keeping pH between 6.0 and 7.5 with repeated additions of a sodium bicarbonate solution.

The mixture is reacted for 40 minutes at +5°C ± 1°C, then the acetone solution is extracted with 3x150 ml of toluene.

### Deformylation

The solution is acidified with concentrated HCl at pH 0.5, stirring for 20 hours at 25°C.

The acid solution is adjusted to pH 1.5 with sodium bicarbonate, washed again with 2x150 ml of toluene and decolourized with 2.5 g of oenological charcoal.

### Osmotic filtration

The aqueous solution, 300 ml, is filtered at 20°C under 38 bars through a nanofiltration membrane having a ≤ 50% retention capacity for sodium chloride.

The mother liquors containing Cefonicid are diluted five times to the starting volume with distilled water and filtered again.

### Freeze-drying

The concentrated solution containing the product in a 30%-40% percentage, is cooled at +10°C ± 2°C and adjusted to pH = 5.2 with sodium bicarbonate. The mixture is decolourized with 2.5 g of oenological charcoal and the solution is frozen at -40°C in a steel tray. The thickness of the frozen solution is 1.5 cm.

The mixture is freeze-dried for 20 hours bringing the temperature from the heating fluid to 45°C in 6 hours and freeze-drying at this temperature for a further 14 hours. The vacuum used is 150 micron. 52 g of freeze-dried disodium Cefonicid are obtained.
K. Fisher = 1.5%
Chemical purity = 97% determined by HPLC
Residual solvents: traces.

### Example 2

The procedure of Example 1 is repeated, with the following changes:
- in the deformylation step, the acid solution is adjusted to pH 5.2 instead of 1.5 with sodium bicarbonate;
- the osmotic filtration is carried out under 35 bars instead of 38 bars;
- in the freeze-drying step, the adjustment of the solution to pH 5,2 is omitted.
54 g of freeze-dried disodium Cefonicid are obtained.
Chemical purity = 98% determined by HPLC
Residual solvents: traces.

### Example 3

The procedure of Example 1 is repeated, with the following changes in the deformylation step:

### Deformylation

The solution is acidified with concentrated HCl at pH = 0.5, stirring for 20 hours at 25°C.

The acid solution is adjusted to pH = 1.5 with sodium bicarbonate, washed again with 2x150 ml of toluene, saturated with 120 g of sodium chloride and extracted with 3x150 ml of acetone. The combined organic extracts are added with 350 ml of H₂O and the acetone phase is extracted with 3x150 ml of toluene.
54 g of freeze-dried disodium Cefonicid are obtained.
Chemical purity = 98% determined by HPLC
Residual solvents: traces.

### Example 4

The procedure of Example 3 is repeated, with the following changes:
- in the deformylation step, the combined organic extracts from the acetone extraction are added with 350 ml of H₂O and the acetone phase are extracted with 3x150 ml of toluene. The acid aqueous solution is adjusted to pH = 5.2 with sodium bicarbonate, washed again with 2x150 ml of toluene and decolourized with 2.5 g of charcoal;
- in the freeze-drying step, the adjustment of the solution to pH 5,2 is omitted.
54 g of freeze-dried disodium Cefonicid are obtained.
Chemical purity = 98% determined by HPLC
Residual solvents: traces.

## Claims

1. A process for the preparation of cephalosporins of formula (I) 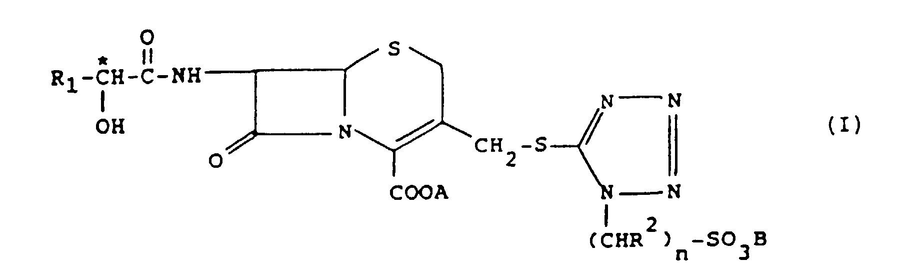 wherein
- R₁ is a phenyl group, optionally substituted with 1 to 3 substituents selected from halogens and hydroxyl groups, a heterocyclic ring of 4 to 7 atoms, both aromatic and non aromatic or a group selected from dihydrophenyl, cyclohexyl, hydroxymethyl, formamido, amido and carboxymethylamino;
- R₂ is hydrogen or a straight or branched alkyl group having 1 to 12 carbon atoms;
- n is an integer of 1 to 10;
- A and B, which are the same or different, are pharmaceutically acceptable cations, selected from cations of alkali or alkaline-earth metals as sodium, potassium and calcium or cations selected from ammonium or the cations of organic amines,
which comprises the following steps:
a) condensation of 7-amino-3-cephem-4-carboxylic acid of formula (II) 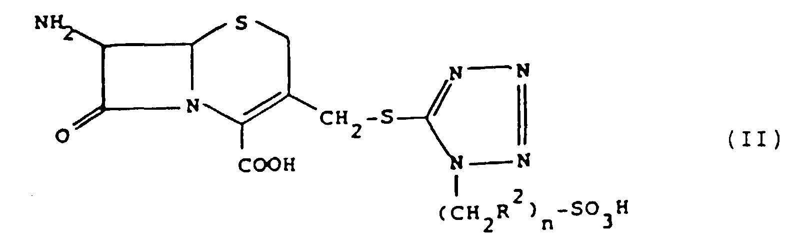 in which R₂ and n have the meaning defined above, with the formyl derivative of formula (III) 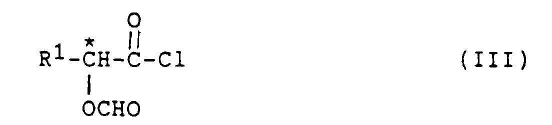 wherein R₁ is as defined above;
the reaction being carried out in an organic or aqueous solvent or in mixtures thereof at pH ranging from 6 to 7.0 by addition of proton acceptors;
b) deformylation of the reaction product in aqueous phase by treatment with an acid at pH 0.5-1.0 to give a product of formula (I) 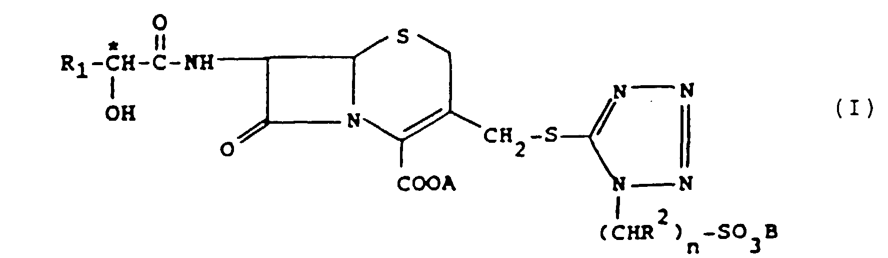
c) adjustment of the aqueous solution to pH ranging from 1.5 to 6.0 with bases containing sodium and osmotic filtration of the solution to remove the excess sodium chloride;
d) adjustment of the osmotic filtrated solution to pH ranging from 1.5 to 6.0 with bases containing sodium and freeze-drying.

2. A process according to claim 1, steps a) and b), in which the product obtained from step b) is recovered with a process which comprises:
e) extraction of the acid product in an organic solvent by salting the aqueous phase;
f) adjustment of the organic phase to pH ranging from 1.5 and 2.5 with bases containing sodium, filtration of the solution and re-extraction of the product in the aqueous phase;
g) adjustment of the aqueous solution to pH ranging from 1.5 to 6.0 with bases containing sodium and osmotic filtration of the solution to remove the excess sodium chloride;
h) optional adjustment to pH 5.2 with bases containing sodium and freeze-drying of the solution.

3. A process according to claim 1 or 2 for the preparation of Cefonicid.

4. A process according to any one of claims 1 to 3, in which step a) is carried out in acetone, methyl acetate, tetrahydrofuran, acetonitrile, water or in a mixture thereof and the proton acceptor is selected from NaHCO₃, NH₃, NAOH, Na₂CO₃ and triethylamine, tetramethylguanidine and diazobicycloundecene.

5. A process according to any one of the above claims, in which step a) is carried out at temperatures from 0°C to +15°C.

6. A process according to any one of the above claims, in which step a) is carried out in a water/acetone mixture in a 30/70 to 70/30 volume ratio, using sodium bicarbonate as proton acceptor.

7. A process according to any one of the above claims, in which the deformylation is performed at pH ranging from 0.5 to 1.5 in an aqueous phase in the presence of organic or inorganic acids selected from HCl, H₂SO₄, H₃PO₄, methanesulfonic acid and trifluoroacetic acid.

8. A process according to claim 6, in which acetone is removed from the acetone/water mixture by extraction with toluene.

9. A process according to any one of the above claims, in which the deformylation is carried out at +20°C to +40°C with hydrochloric acid at pH = 0.5.

10. A process according to any one of the above claims in which step c) is carried out adjusting the solution to pH values ranging from 1.5 to 6.0 with NaHCO₃, Na₂CO₃ or NaOH and the solution is filtered through a nanofiltration membrane at temperatures ranging from 0°C to +50°C under a pressure of 10-50 x 10⁵ Pa.

11. A process as claimed in claim 10, in which the pH of the aqueous solution is adjusted to 1.5 or 5.2 and the base used is sodium bicarbonate.

12. A process as claimed in claim 10, in which the aqueous solution containing the product is filtered through a nanofiltration membrane having a ≤ 50% retention capacity for sodium chloride at a temperature from 20°C and under a pressure of 38 x 10⁵ Pa.

13. A process as claimed in claim 1, in which step d) is carried out adjusting-the pH of the solution to 4.5-6.5 with NaHCO₃ or NaOH at a temperature from 0°C to +50°C and the freeze-drying is performed freezing the solution at -40°C to -60°C and subsequently heating the fluid at +40°C and +70°C and under a freeze-drying vacuum below 39.9966 Pa (300 µm).

14. A process according to claim 13, in which pH of the solution is adjusted to 5.2 with sodium bicarbonate.

15. A process according to claim 13, in which the adjustment of pH is carried out at +10°C.

16. A process according to claim 2, in which step f) is carried out adding water to the organic phase containing the active ingredient and removing the organic phase by means of extraction with solvents selected from toluene, ethyl acetate, methyl isobutyl ketone, methylene chloride, chloroform.

17. A process according to claim 2, in which step g) is carried out by adjustment of the pH of the aqueous solution to values ranging from 1.5 to 6.0 with bases selected from NaHCO₃ and NaOH and filtration of the solution through a nanofiltration membrane at temperatures ranging from 0° to +50°C and under pressures of 10-40 x 10⁵ Pa.

18. A process according to claim 17, in which the pH of the aqueous solution is adjusted to 1.5 or 5.2 and the base used is sodium bicarbonate.

19. A process according to claim 18, in which the aqueous solution containing the product is filtered through a nanofiltration membrane having a ≤ 50% retention capacity for sodium chloride at a temperature from 20°C and under a pressure of 38 x 10⁵ Pa.

20. A process according to claim 2, in which step g) is carried out adjusting the pH of the solution to 4.5-6.5 with NaHCO₃, Na₂CO₃ or NaOH at a temperature from 0° to +50°C and the freeze-drying is performed freezing the solution at -40°C to -60°C, subsequently heating the fluid at +40°C to +70°C and under a freeze-drying vacuum below 39.9966 Pa (300 µm).

## Patentansprüche

1. Verfahren zur Herstellung von Cephalosporinen der Formel (I) 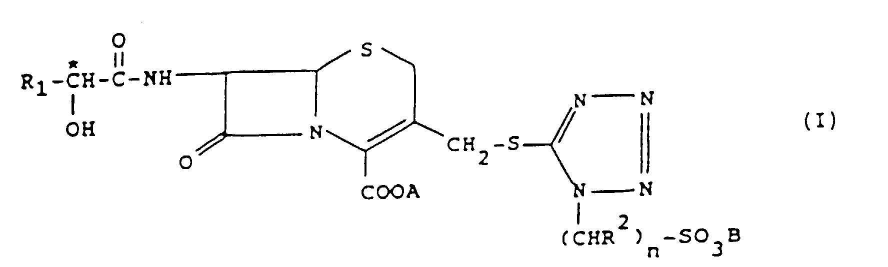 worin bedeuten:
R₁ eine Phenylgruppe, die gegebenenfalls durch 1 bis 3 Substituenten, ausgewählt aus Halogenen und Hydroxylgruppen, substituiert ist, einen heterocyclischen Ring mit 4 bis 7 Atomen, der sowohl aromatisch als auch nicht-aromatisch sein kann, oder eine Gruppe, ausgewählt aus Dihydrophenyl, Cyclohexyl, Hydroxymethyl, Formamido, Amido und Carboxymethylamino;
R₂ Wasserstoff oder eine geradkettige (unverzweigte) oder verzweigte Alkylgruppe mit 1 bis 12 Kohlenstoffatomen;
n eine ganze Zahl von 1 bis 10;
A und B, die gleich oder verschieden sind, pharmazeutisch akzeptable Kationen, ausgewählt aus den Kationen von Alkali- oder Erdalkalimetallen wie Natrium, Kalium und Calcium, oder Kationen, ausgewählt aus Ammonium oder den Kationen von organischen Aminen,
das die folgenden Stufen umfaßt:
a) Kondensieren einer 7-Amino-3-cephem-4-carbonsäure der Formel (II) 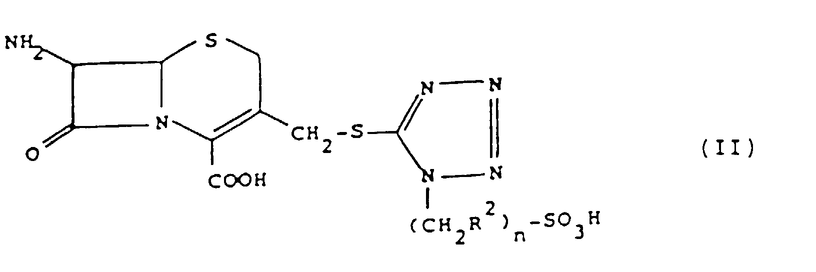 in der R₂ und n die oben angegeben Bedeutungen haben,
mit dem Formyl-Derivat der Formel (III) 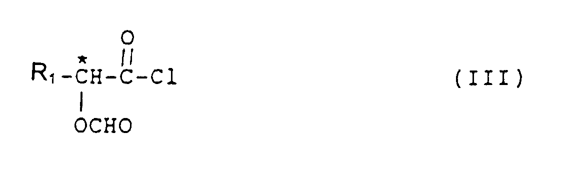 worin R₁ wie oben definiert ist,
wobei die Reaktion in einem organischen oder wäßrigen Lösungsmittel oder in einer Mischungen davon bei einem pH-Wert in dem Bereich von 6 bis 7,0 unter Zugabe von Protonen-Akzeptoren durchgeführt wird;
b) Deformylieren des Reaktionsprodukts in wäßriger Phase durch Behandlung mit einer Säure bei einem pH-Wert von 0,5 bis 1,0 unter Bildung eines Produktes der Formel (I) 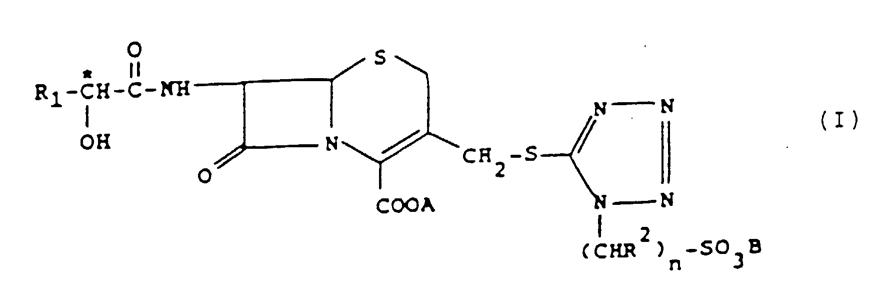
c) Einstellen der wäßrigen Lösung auf einen pH-Wert in dem Bereich von 1,5 bis 6,0 mit Basen, die Natrium enthalten, und osmotisches Filtrieren der Lösung zur Entfernung des überschüssigen Natriumchlorids; und
d) Einstellen der osmotisch filtrierten Lösung auf einen pH-Wert in dem Bereich von 1,5 bis 6,0 mit Basen, die Natrium enthalten, und Gefriertrocknen.

2. Verfahren nach Anspruch 1, Stufen (a) und (b), bei dem das in der Stufe (b) erhaltene Produkt nach einem Verfahren gewonnen wird, das umfaßt:
e) das Extrahieren des Säureprodukts in einem organischen Lösungsmittel durch Aussalzen der wäßrigen Phase;
f) das Einstellen der organischen Phase auf einen pH-Wert in dem Bereich von 1,5 bis 2,5 mit Basen, die Natrium enthalten, das Filtrieren der Lösung und das Rückextrahieren des Produkts in die wäßrige Phase;
g) das Einstellen der wäßrigen Lösung auf einen pH-Wert in dem Bereich von 1,5 bis 6,0 mit Basen, die Natrium enthalten, und das osmotische Filtrieren der Lösung zur Entfernung des überschüssigen Natriumchlorids; und
h) gegebenenfalls das Einstellen auf pH 5,2 mit Basen, die Natrium enthalten, und das Gefriertrocknen der Lösung.

3. Verfahren nach Anspruch 1 oder 2 zur Herstellung von Cefonicid.

4. Verfahren nach einem der Ansprüche 1 bis 3, bei dem die Stufe (a) in Aceton, Methylacetat, Tetrahydrofuran, Acetonitril, Wasser oder in einer Mischung davon durchgeführt wird und der Protonenakzeptor ausgewählt wird aus NaHCO₃, NH₃, NaOH, Na₂CO₃ und Triethylamin, Tetramethylguanidin und Diazobicycloundecen.

5. Verfahren nach einem der vorhergehenden Ansprüche, bei dem die Stufe (a) bei Temperaturen von 0 bis +15°C durchgeführt wird.

6. Verfahren nach einem der vorhergehenden Ansprüche, bei dem die Stufe (a) in einem Wasser/Aceton-Gemisch mit einem Volumenverhältnis von 30/70 bis 70/30 unter Verwendung von Natriumbicarbonat als Protonenakzeptor durchgeführt wird.

7. Verfahren nach einem der vorhergehenden Ansprüche, bei dem das Deformylieren bei einem pH-Wert in dem Bereich von 0,5 bis 1,5 in einer wäßrigen Phase in Gegenwart von organischen oder anorganischen Säuren, ausgewählt aus HCI, H₂SO₄, H₃PO₄, Methansulfonsäure und Trifluoressigsäure, durchgeführt wird

8. Verfahren nach Anspruch 6, bei dem Aceton durch Extrahieren mit Toluol aus dem Aceton/Wasser-Gemisch entfernt wird.

9. Verfahren nach einem der vorhergehenden Ansprüche, bei dem das Deformylieren bei +20°C bis +40°C mit Chlorwasserstoffsäure bei einem pH-Wert von 0,5 durchgeführt wird.

10. Verfahren nach einem der vorhergehenden Ansprüche, bei dem die Stufe (c) durchgeführt wird durch Einstellen der Lösung auf pH-Werte in dem Bereich von 1,5 bis 6,0 mit NaHCO₃, Na₂CO₃ oder NaOH und bei dem die Lösung durch eine Nanofiltrationsmembran bei Temperaturen in dem Bereich von 0°C bis +50°C unter einem Druck von 10 bis 50 x 10⁵ Pa filtriert wird.

11. Verfahren nach Anspruch 10, bei dem der pH-Wert der wäßrigen Lösung auf 1,5 oder 5,2 eingestellt wird und als Base Natriumbicarbonat verwendet wird.

12. Verfahren nach Anspruch 10, bei dem die das Produkt enthaltende wäßrige Lösung durch eine Nanofiltrationsmembran mit einem Rückhaltevermögen für Natriumchlorid von ≤ 50 % filtriert wird bei einer Temperatur von 20°C und unter einem Druck von 38 x 10⁵ Pa.

13. Verfahren nach Anspruch 1, bei dem die Stufe (d) durchgeführt wird durch Einstellen des pH-Wertes der Lösung auf 4,5 bis 6,5 mit NaHCO₃ oder NaOH bei einer Temperatur von 0 bis +50°C und bei dem das Gefriertrocknen durchgeführt wird durch Einfrieren der Lösung auf -40 bis -60°C und anschließendes Erhitzen der Flüssigkeit auf +40°C und +70°C und unter einem Gefriertrocknungs-Vakuum von < 39,9966 Pa (< 300 µm).

14. Verfahren nach Anspruch 13, bei dem der pH-Wert der Lösung mit Natriumbicarbonat auf 5,2 eingestellt wird.

15. Verfahren nach Anspruch 13, bei dem das Einstellen des pH-Wertes bei +10°C durchgeführt wird.

16. Verfahren nach Anspruch 2, bei dem die Stufe (f) durchgeführt wird durch Zugabe von Wasser zu der organischen Phase, die den aktiven Bestandteil enthält, und durch Enffernen der organischen Phase durch Extrahieren mit Lösungsmitteln, ausgewählt aus der Gruppe Toluol, Ethylacetat, Methylisobutylketon, Methylenchlorid und Chloroform.

17. Verfahren nach Anspruch 2, bei dem die Stufe (g) durchgeführt wird durch Einstellen des pH-Wertes der wäßrigen Lösung auf Werte in dem Bereich von 1,5 bis 6,0 mit Basen, die ausgewählt werden aus NaHCO₃ und NaOH, und durch Filtrieren der Lösung durch eine Nanofiltrations-Membran bei Temperaturen in dem Bereich von 0 bis +50°C und unter Drucken von 10 - 40 x 10⁵ Pa.

18. Verfahren nach Anspruch 17, bei dem der pH-Wert der wäßrigen Lösung auf 1,5 oder 5,2 eingestellt wird und als Base Natriumbicarbonat verwendet wird.

19. Verfahren nach Anspruch 18, bei dem die das Produkt enthaltende wäßrige Lösung durch eine Nanofiltrationsmembran filtriert wird, die ein Rückhaltevermögen für Natriumchlorid von ≤ 50 % aufweist bei einer Temperatur von 20°C und unter einem Druck von 38 x 10⁵ Pa.

20. Verfahren nach Anspruch 2, bei dem die Stufe (g) durchgeführt wird durch Einstellen des pH-Wertes der Lösung auf 4,5 bis 6,5 mit NaHCO₃, Na₂CO₃ oder NaOH bei einer Temperatur von 0 bis +50°C und bei dem das Gefriertrocknen durchgeführt wird durch Einfrieren der Lösung bei -40 bis -60°C, anschließendes Erhitzen der Flüssigkeit auf +40 bis +70°C und unter einem Gefriertrocknungs-Vakuum von < 39,9966 Pa (< 300 µm).

## Revendications

1. Procédé pour la préparation de céphalosporines selon la formule (I) : 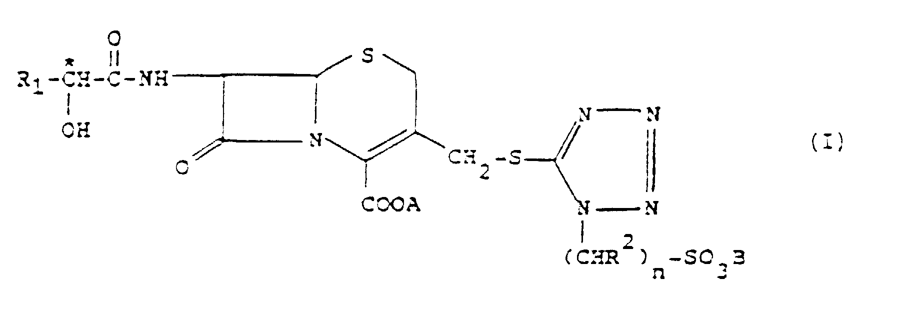 dans laquelle
- R₁ est un groupe phényle facultativement substitué avec 1 à 3 substituants choisis parmi des halogènes et des hydroxyles. un anneau hétérocyclique de 4 à 7 atomes de carbone, à la fois aromatique et non aromatique ou un groupe choisi parmi le dihydrophenyle, le cyclohexyle, le methoxy, le formamide, l'amide et le carboxyméthylamine.
- R₂ est de l'hydrogène ou un groupe alkyle linéaire ou ramifié, ayant entre 1 et 12 atomes de carbone
- n est un nombre entier de 1 à 10;
- A et B, qui sont pareils ou différents, sont des cations acceptables sur le plan pharmaceutique, choisis parmi des cations de métaux alcalins ou alcalinoterreux. tels que le sodium, le potassium et le caicium, au des cations choisis parmi l'ammonium ou les cations d'amines organiques;
qui comporte les étapes suivantes :
a) la condensation d'acide 7-amino-3-céphem-4-carboxylique selon la formule (II) 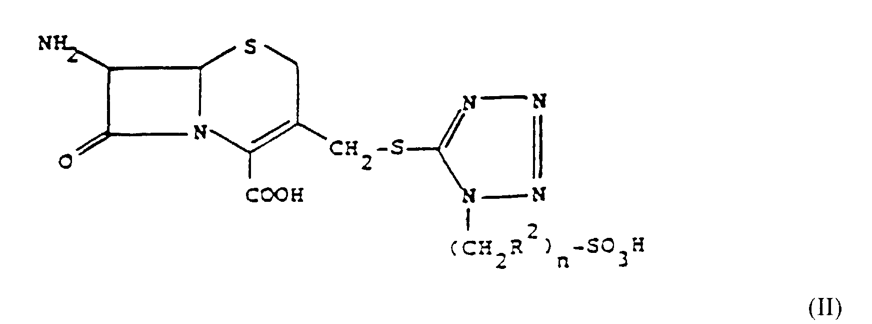 dans laquelle R₂ et n ont la signification définie ci-dessus avec le dérivé de formyle selon la formule (III). 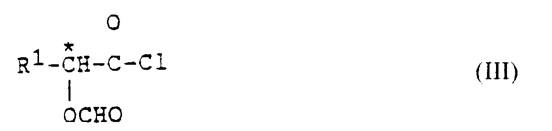 dans laquelle R₁ est comme défini ci-dessus;
la réaction s'effectuant dans un solvant organique ou aqueux ou dans des mélanges de celui-ci à un pH allant de 6 à 7,0 par adjonction d'accepteurs de protons;
b) la déformylation du produit de la réaction dans une phase aqueuse par le traitement avec un acide à pH 0,5-1,0 pour obtenir un produit selon la formule (I) 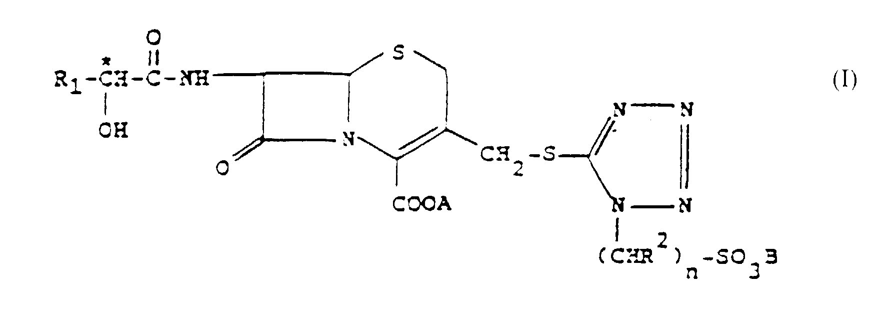
c) l'ajustage de la solution aqueuse à un pH allant de 1,5 à 6,0 avec des bases contenant du sodium et la filtration osmotique de la solution pour enlever l'excès de chlorure de sodium;
d) l'ajustage de la solution ayant subi la filtration osmotique à un pH allant de 1,5 à 6,0 avec des bases contenant du sodium et la lyophilisation.

2. Procédé selon la revendication 1, étapes a) et b) caractérisé en ce que le produit obtenu de l'étape b) est récupéré au moyen d'un procédé qui comporte :
e) l'extraction du produit acide dans un solvant organique par le salage de la phase aqueuse;
f) l'ajustage de la phase organique à un pH allant de 1,5 à 2,5 avec des bases contenant du sodium, la filtration de la solution et la réextraction du produit dans la phase aqueuse.
g) l'ajustage de la solution aqueuse à un pH allant de 1,5 à 6,0 avec des bases contenant du sodium, la filtration osmotique de la solution pour enlever l'excès de chlorure de sodium;
h) l'ajustage facultatif à un pH de 5,2 avec des bases contenant du sodium et la lyophilisation de la solution.

3. Procédé selon la revendication 1 ou 2, pour la préparation de Céfonicide.

4. Procédé selon une des revendications 1 à 3, caractérisé en ce que l'étape a) s'effectue dans de l'acétone, de l'acétate de méthyle, du tétrahydrofurane, de l'acétonitrile. de l'eau ou dans un mélange de ceux-ci et en que l'accepteur de protons est choisi parmi NaHCO₃, NH₃, NaOH, Na₂CO₃, le triéthylamine, le tétraméthylguanidine, le diazobicycloundécène.

5. Procédé selon une des revendications ci-dessus, caractérisé en ce que l'étape a) s'effectue à des températures allant de 0°C à +15°C.

6. Procédé selon une des revendications ci-dessus, caractérisé en ce que l'étape a) s'effectue dans un mélange eau/acétone dans un rapport de 30/70 à 70/30 en volume, en utilisant du bicarbonate de sodium comme accepteur de protons.

7. Procédé selon une des revendications ci-dessus, caractérisé en ce que la déformylation s'effectue à un pH allant de 0,5 à 1,5 dans une phase aqueuse en présence d'acides organiques ou inorganiques choisis parmi HC₁, H₂SO₄, H₃PO₄, l'acide méthanesulfonique et l'acide trifluoroacétique.

8. Procédé selon la revendication 6, caractérisé en ce que l'on enlève l'acétone du mélange acétone/eau par extraction avec du toluène.

9. Procédé selon une des revendications ci-dessus, caractérisé en ce que la déformylation est réalisée à une température entre +20°C et +40°C avec de l'acide chlorhydrique à pH = 0,5.

10. Procédé selon une des revendications ci-dessus, caractérisé en ce que l'étape c) est réalisée en ajustant la solution à des valeurs de pH allant de 1,5 à 6,0 avec NaHCO₃, Na₂CO₃ ou NaOH et en ce que la solution est filtrée à travers une membrane de nanofiltration à des températures allant de 0°C à -50°C et sous une pression de 10-50 x 10⁵ Pa.

11. Procédé selon la revendication 10, caractérisé en ce que l'on ajuste le pH de la solution aqueuse à 1,5 ou 5,2 et en ce que la base utilisée est du bicarbonate de sodium.

12. Procédé selon la revendication 10, caractérisé en ce que la solution aqueuse contenant le produit est filtrée à travers une membrane de nanofiltration ayant une capacité de rétention ≤ 50% pour le chlorure de sodium à une température de 20°C et sous une pression de 38 x 10⁵ Pa.

13. Procédé selon la revendication 1, caractérisé en ce que l'étape d) est réalisée en ajustant le pH de la solution à 4,5-6,5 avec NaHCO₃ ou NaOH à une température allant de 0°C à +50°C et en ce que la lyophilisation est réalisée pour geler la solution à une température entre -40°C et -60°C suivi du réchauffement du fluide à une température entre +40°C et +70°C et sous un vide de lyophilisation en dessous de 39.9966 Pa (300 µm).

14. Procédé selon la revendication 13, caractérisé en ce que l'on ajuste le pH de la solution à 5,2 avec du bicarbonate de sodium.

15. Procédé selon la revendication 13, caractérisé en ce que l'ajustage du pH est réalisé à une température de +10°C.

16. Procédé selon la revendication 2, caractérisé en ce que l'étape f) est réalisée en rajoutant de l'eau à la phase organique contenant l'ingrédient actif et en enlevant la phase organique par une extraction à l'aide de solvants choisis parmi le toluène. l'acétate d'éthyle, le méthylisobutylcétone, le chlorure de méthylène, le chloroforme.

17. Procédé selon la revendication 2, caractérisé en ce que l'étape g) est réalisée en ajustant le pH de la solution aqueuse à des valeurs allant de 1,5 à 6,0 avec des bases choisies parmi NaHCO₃ et NaOH et par la filtration de la solution à travers une membrane de nanofiltration à des températures allant de 0°C à +50°C et sous une pression de 10-40 x 10⁵ Pa.

18. Procédé selon la revendication 17, caractérisé en ce que l'on ajuste le pH de la solution aqueuse à 1,5 ou 5,2 et en ce que la base utilisée est du bicarbonate de sodium.

19. Procédé selon la revendication 18, caractérisé en ce que la solution aqueuse contenant le produit est filtrée à travers une membrane de nanofiltration ayant une capacité de rétention ≤ 50% pour le chlorure de sodium à une température de 20°C et sous une pression de 38 x 10⁵ Pa.

20. Procédé selon la revendication 2, caractérisé en ce que l'étape g) est réalisée en ajustant le pH de la solution à 4,5-6,5 avec NaHCO₃, Na₂CO₃ ou NaOH à une température de 0° à +50°C et en ce que la lyophilisation est réalisée pour geler la solution à une température entre -40°C et -60°C suivi du réchauffement du fluide à une température entre +40°C et +70°C et sous un vide de lyophilisation en dessous de 39.9966 Pa (300 µm).
